(19)

Europäisches
Patentamt
European
Patent Office
Office européen
des brevets

(11) **EP 3 928 831 B1**

(12) **EUROPEAN PATENT SPECIFICATION**

(45) Date of publication and mention
of the grant of the patent:
**06.08.2025 Bulletin 2025/32**

(21) Application number: **19915947.6**

(22) Date of filing: **19.02.2019**

(51) International Patent Classification (IPC):
**A61N 5/06** *(2006.01)*      **G16H 20/40** *(2018.01)*

(52) Cooperative Patent Classification (CPC):
**A61N 5/062; G16H 20/40; G16H 50/20**

(86) International application number:
**PCT/JP2019/006078**

(87) International publication number:
**WO 2020/170330 (27.08.2020 Gazette 2020/35)**

(54) **METHOD FOR DETERMINING CONDITION PARAMETERS FOR PHOTODYNAMIC THERAPY AND PHOTODYNAMIC THERAPY APPARATUS**

VERFAHREN ZUR BESTIMMUNG VON ZUSTANDSPARAMETERN FÜR DIE PHOTODYNAMISCHE THERAPIE UND PHOTODYNAMISCHE THERAPIEVORRICHTUNG

PROCÉDÉ DE DÉTERMINATION DE PARAMÈTRES D'ÉTAT POUR THÉRAPIE PHOTODYNAMIQUE ET APPAREIL DE THÉRAPIE PHOTODYNAMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**29.12.2021 Bulletin 2021/52**

(73) Proprietor: **Otsuka Electronics Co., Ltd.
Hirakata-shi, Osaka 573-1132 (JP)**

(72) Inventors:
• **KOHASHI, Masayuki
  Hirakata-shi, Osaka 573-1132 (JP)**
• **OTA, Kazuhide
  Hirakata-shi, Osaka 573-1132 (JP)**

• **HARADA, Yasuo
  Osaka-shi, Osaka 540-0021 (JP)**

(74) Representative: **Eisenführ Speiser
Patentanwälte Rechtsanwälte PartGmbB
Postfach 10 60 78
28060 Bremen (DE)**

(56) References cited:
WO-A1-2004/112902    WO-A1-2015/125732
JP-A- 2015 522 305    JP-A- 2017 006 454
KR-B1- 101 254 752    US-A1- 2004 147 501
US-A1- 2005 112 131    US-A1- 2006 282 132
US-A1- 2014 128 799    US-A1- 2016 361 418
US-A1- 2017 042 938

EP 3 928 831 B1

# EP 3 928 831 B1

## Description

### TECHNICAL FIELD

The present invention relates to photodynamic therapy using 5-aminolevulinic acids.

### BACKGROUND

US 2006/0282132 A1 provides PDT equipment which can absolutely treat the deep lesioned part with preserving the superficial membrane of the healthy region and discloses photodynamic therapy equipment for treating lesioned part by using a photosensitive substance, which is activated by the light having a peak intensity of a predetermined range but is almost not activated by the light having the peak intensity out of the predetermined range, equipped with an irradiation means irradiating into the body a pulsed light of the wavelength having the potential for activating the photosensitive substance, and a control means controlling the peak intensity of the light irradiated by the irradiation means, and the control means allows the irradiation means to irradiate the light having the high peak intensity in order that the light arriving at the deep-lying lesioned part is to achieve the peak intensity of the predetermined range.

In order to provide a preventive or therapeutic composition for a therapy-resistant cancer having therapy-resistant cancer cells, a preventive or therapeutic composition for a therapy-resistant cancer having therapy-resistant cancer cells is proposed in US 2016/0361418 A1, which is for use in photodynamic therapy, wherein the composition comprises ALAs.

US 2005/0112131 A1 relates to the enhancement of radiation sensitivity by using photodynamic therapy.

US 2017/0042938 A1 relates to the use of a photoactivatable Porphyrin-Derivative in extracorporeal photophoresis (ECP) treatment, in which a patient's blood of part of it containing said Porphyrin-derivative is/are exposed to light of a wavelength which activates said photoactivatable Porphyrin-Derivative.

Photodynamic therapy (PDT) is therapy that utilizes the killing effect of reactive oxygen species including singlet oxygen generated by excitation by administering a photosensitizer or its precursor, causing the photosensitizer to accumulate in affected areas such as tumor tissues, newborn blood vessels, and skin surfaces, and irradiating a light beam corresponding to an absorption band wavelength of the photosensitizer as excitation light.

5-aminolevulinic acids (5-ALA) are oral absorbability and metabolized to protoporphyrin IX (PpIX) in the process of biosynthesis of heme in intracellular mitochondria. PpIX is a photosensitizer that has an absorption band near 410 nm, called a Soret band, and an absorption band near 500 to 650 nm, called a Q band, and generates reactive oxygen species by irradiation with light having these absorption band wavelengths, and is used for PDT.

Regarding PDT using 5-ALAs, Patent Document 1 describes that the intracellular accumulation of PpIX biosynthesized from 5-ALA increased in a 5-ALA concentration-dependent manner, and the sensitivity of the cells to light irradiation increased, that is, the cells were likely to die. In addition to the intracellular PpIX accumulation, it is also known in the art that the effect of PDT depends on light irradiation energy density, which is an integrated value of the light illuminance and the irradiation time (Patent Document 2).

As described above, while it is known that there is a tendency to depend on these factors, an exact relationship between the intracellular PpIX accumulation and the light irradiation energy density toward the killing effect in PDT has not been clarified so far. In addition, any relationship or similarity between these dependencies on the killing effect in different target cell types, origins, and primary diseases is not known beyond the types and origins of the target cell and the like.

In addition, Patent Documents 3 and 4, and Non-Patent Document 1 relate to PDT, but fail to disclose any strict relationship between the intracellular PpIX accumulation and the light irradiation energy density toward the killing effect in PDT.

### PATENT DOCUMENT

Patent Document 1: WO 2015/125732 A
Patent Document 2: JP 2017-526371 A
Patent Document 3: JP 5612246 B2
Patent Document 4: US 6,609,014

### NON-PATENT DOCUMENT

Non-Patent Document 1: Ouyang G. et al., "Inhibition of autophagy potentiates the apoptosis-inducing effects of photodynamic therapy on human colon cancer cells", Photodiagnosis Photodyn Ther. 2018; 21: 396-403

# EP 3 928 831 B1

## SUMMARY

In PDT, excessive light irradiation kills normal cells and leads to increased side effects, while under-irradiation cannot sufficiently kill target cells.

It is an object of the present invention to provide a method for determining condition parameters for optimized photodynamic therapy by accurately determining the condition parameters of photodynamic therapy.

It is also an object of the present invention to provide a photodynamic therapy apparatus using such a method for determining condition parameters for photodynamic therapy.

In the present invention, it has been found that cell death is curvilinearly regressed by a certain regression curve, for example, a four-parameter logistic model, depending on the intracellular PpIX accumulation and the light irradiation energy density regardless of cell species. Thus, it is possible to determine a proper amount of light irradiation energy and a dose of photosensitizer, and to predict a therapeutic effect, in a target disease where PpIX accumulation is observed.

Thus, the present invention provides a method for determining condition parameters for photodynamic therapy in which after administration of 5-aminolevulinic acids, protoporphyrin IX accumulated in cells is irradiated with light, the method including steps of:

> calculating, by a processor in an experimental phase, a regression curve representing a correlation among three condition parameters: cell viability (Y), intracellular protoporphyrin IX accumulation (X), and light irradiation energy density (P); and
>
> selecting in advance, by the processor prior to initiation of therapy, two condition parameters from the cell viability (Y), the intracellular protoporphyrin IX accumulation (X), and the light irradiation energy density (P), and then determining the remaining condition parameter using the regression curve.

Further, a photodynamic therapy apparatus according to the present invention includes:

> a light source for irradiating protoporphyrin IX accumulated in cells with light after administration of 5-aminolevulinic acids;
> a light intensity control unit for controlling light irradiation energy density (P);
> a data input unit for inputting data;
> a processor for calculating data; and
> a data storage unit for recording data,
> wherein the processor calculates, in an experimental phase, a regression curve representing a correlation among three condition parameters of cell viability (Y), intracellular protoporphyrin IX accumulation (X), and the light irradiation energy density (P), and
> the processor selects in advance, prior to initiation of therapy, two condition parameters from the cell viability (Y), the intracellular protoporphyrin IX accumulation (X), and the light irradiation energy density (P), and determines the remaining condition parameter using the regression curve.

It is preferable that the regression curve is expressed by the following model equation using four parameters A to D.

$$Y = (A-D)/(1+(X/C)^B)+D$$

It is preferable that the cell has a protoporphyrin IX-accumulating disease selected from a group consisting of leukemia, tumor, cancer, viral infection, skin disease, eye disease, autoimmune disease, and graft-versus-host disease (GVHD).

The present invention also relates to a photodynamic therapy composition including 5-aminolevulinic acids having a dose corresponding to the intracellular protoporphyrin IX accumulation (X) determined by the above-described method for determining condition parameters for photodynamic therapy.

The present invention also provides a method for determining light irradiation conditions in photodynamic therapy in which a subject is light-irradiated to inactivate cells with protoporphyrin IX accumulation after administration of 5-aminolevulinic acids to the subject, the method including steps of:

> measuring, by a processor, an accumulation of protoporphyrin IX in cells from the subject; and
> determining, by the processor, light irradiation energy density using a predetermined relational expression based on the accumulation of protoporphyrin IX measured in the above step.

It is preferable that said relational expression is determined based on a regression curve representing a correlation among the three condition parameters of cell viability (Y), intracellular protoporphyrin IX accumulation (X), and light irradiation energy density (P), which are calculated in advance.

It is preferable that said relational expression includes a lookup table illustrating a relationship among the protoporphyrin IX accumulation (X) and the light irradiation energy density (P).

It is preferable to determine a plurality of times of light irradiation when the measured protoporphyrin IX accumulation is less than a predetermined value.

The method for determining the condition parameters for photodynamic therapy according to the present invention also preferably includes steps of:

measuring, by a processor, cell viability of a subject after performing therapy using condition parameters determined using said regression curve, and estimating an intracellular protoporphyrin IX accumulation based on the obtained cell viability; and

resetting, by the processor prior to initiation of the next therapy, the light irradiation energy density and the 5-aminolevulinic acid dose using the estimated intracellular protoporphyrin IX accumulation.

In the present invention, the 5-aminolevulinic acids (5-ALAs) include 5-aminolevulinic acid (5-ALA) or a derivative thereof, or a salt thereof. Various administration forms such as intravenous injection, drip infusion, oral administration, transdermal administration, suppository, and intravesical injection can be adopted for administration of such 5-aminolevulinic acids.

EFFECT OF INVENTION

A problem in photodynamic therapy is that excessive light irradiation kills normal cells and leads to increased side effects, while under-irradiation cannot sufficiently kill target cells. Therefore, by adapting the results of regression analysis according to the present invention, light irradiation energy density or a dose of the photosensitizer that appropriately induces cell death can be determined from an intracellular protoporphyrin IX accumulation in target cells of the disease to be treated (leukemia or the like).

In addition, upon calculating an effective protoporphyrin IX accumulation from a correlation between the protoporphyrin IX accumulation and a killing effect, a cancer cell population out of the effective range of protoporphyrin IX accumulation can be calculated to estimate an amount of irradiation, irradiation times, and the like for finally achieving a clinical effect.

BRIEF DESCRIPTION OF THE DRAWINGS

FIG. 1 is a block diagram illustrating an example of a photodynamic therapy apparatus according to the present invention.

FIG. 2 is a flowchart illustrating an example of a method for determining condition parameters for photodynamic therapy according to the present invention.

FIG. 3 is a graph illustrating an example of regression analysis results for four cell lines derived from human adult T-cell leukemia/lymphoma.

FIG. 4 is a graph illustrating an example of a regression analysis result for Jurkat cells derived from human T-cell leukemia/lymphoma.

DESCRIPTION OF EMBODIMENT

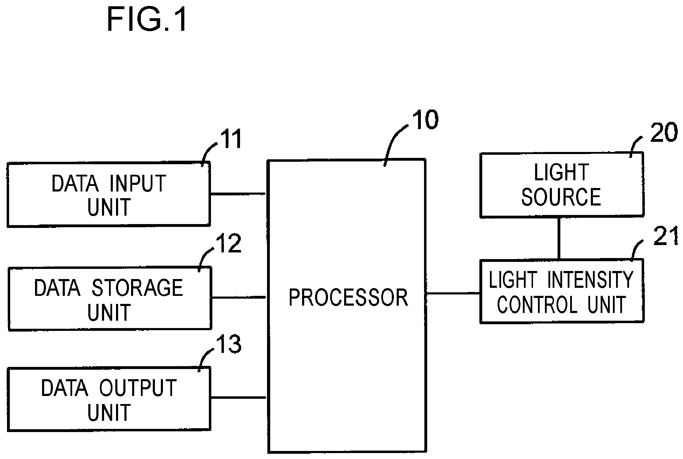

FIG. 1 is a block diagram illustrating an example of a photodynamic therapy apparatus according to the present invention. A photodynamic therapy apparatus includes a light source 20, a light intensity control unit 21, a processor 10 that calculates data, a data input unit 11 that inputs data, a data storage unit 12 that stores data, a data output unit 13 that outputs data, and the like.

The light source 20 has a function of irradiating protoporphyrin IX (PpIX) accumulated in cells with light after administering 5-aminolevulinic acids (5-ALAs) to a subject. The wavelength of light is set to an absorption wavelength of PpIX, for example, 410 nm, 545 nm, 580 nm, and 630 nm. The light intensity control unit 21 controls light irradiation energy density (P) of the light emitted from the light source 20.

The processor 10 performs control of the entire apparatus, data processing, and the like according to a preset program. The data input unit 11 includes a manual input device, such as a keyboard, a touch panel, and a mouse, or a remote input device by wired communication or wireless communication. The data storage unit 12 includes a semiconductor memory, a hard disk, and an optical disk. The data output unit 13 includes a display and a printer.

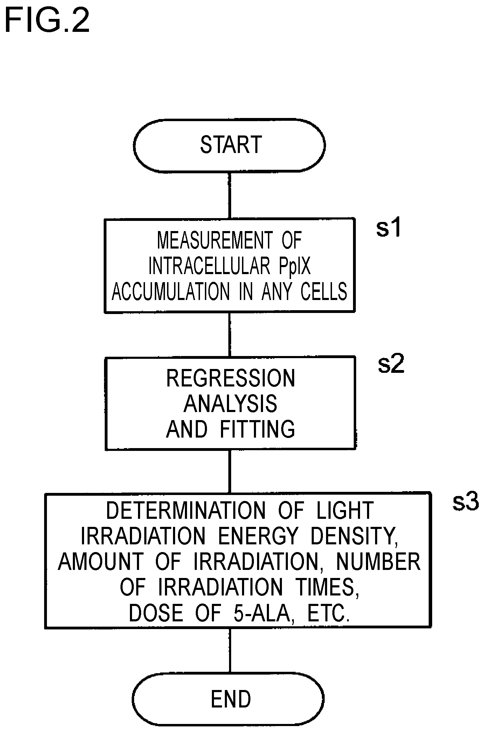

FIG. 2 is a flowchart illustrating an example of a method for determining condition parameters for photodynamic therapy according to the present invention. First, in step s1, in an experimental phase, 5-ALAs having various doses are administered to the subject, and then, an amount of PpIX accumulated in the cells is measured. As this measurement method, for example, a fluorescence detection high-performance liquid chromatography method, and a flow cytometer can be used. The measured intracellular PpIX accumulation is input through the data input unit 11 and stored in the data storage unit 12.

Next, in step s2, cells having various amounts of PpIX accumulation are irradiated with light at various light irradiation energy densities (joules per unit area), and viabilities of individual cells are measured. The various light irradiation energy densities used and the measured viabilities of the individual cells are input through the data input unit 11 and stored in the data storage unit 12. Next, the processor 10 performs regression analysis and fitting on the three condition parameters of cell viability (Y), intracellular PpIX accumulation (X), and light irradiation energy density (P) which are stored in the data storage unit 12 according to predetermined statistical analysis software to calculate a regression curve representing a correlation among the condition parameters. Any model equation can be assumed for the regression curve, but it is preferable to express the regression curve by the following model equation (1) using four parameters A to D. The calculated regression curve and the parameters A to D defining the regression curve can be stored in the data storage unit 12 and displayed on the data output unit 13.

$$Y = (A-D)/(1+(X/C)^B)+D \quad \cdots (1)$$

Next, in step s3, prior to initiation of therapy, two condition parameters are selected in advance from cell viability (Y), intracellular PpIX accumulation (X), and light irradiation energy density (P) through the data input unit 11, and the processor 10 determines the remaining condition parameter using the regression curve stored in the data storage unit 12. The two condition parameters selected in advance and the determined condition parameter can be stored in the data storage unit 12 and displayed on the data output unit 13. The light irradiation may be performed not only once but also divided into twice or more. In this case, the sum of the energy densities of the respective irradiation times is equal to the light irradiation energy density (P).

For example, 1) in a case the cell viability (Y) and the intracellular PpIX accumulation (X) are selected in advance, the light irradiation energy density (P) can be determined using the regression curve. The determined light irradiation energy density (P) is supplied to the light intensity control unit 21 to control both the amount of irradiation and the number of irradiation times of light emitted from the light source 20.

In addition, 2) in a case the cell viability (Y) and the light irradiation energy density (P) are selected in advance, the intracellular PpIX accumulation (X) can be determined using the regression curve. In this case, a photodynamic therapy composition containing 5-aminolevulinic acids having a dose corresponding to the intracellular PpIX accumulation (X) thus determined can be produced.

3) In a case the intracellular PpIX accumulation (X) and the light irradiation energy density (P) are selected in advance, the cell viability (Y) can be determined using the regression curve.

When a regression curve is used, such a mathematical expression may be used, but a lookup table obtained by converting correlation between the condition parameters P, X, and Y into a database in a digital format may also be used.

(Example 1)

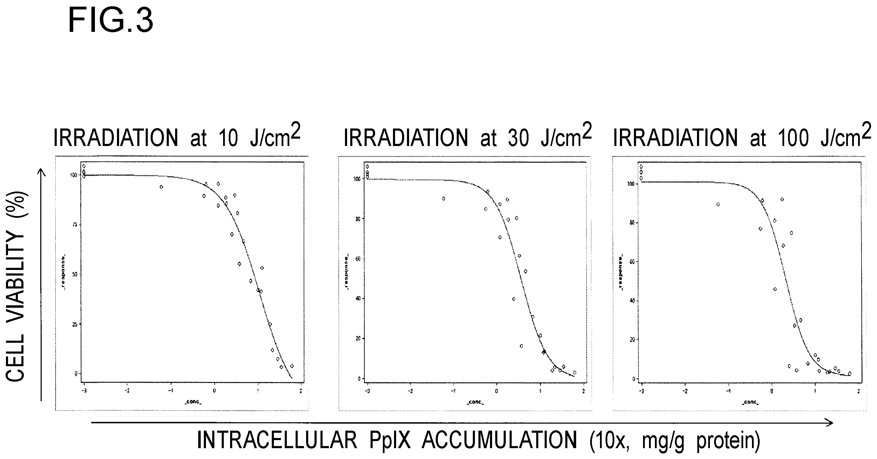

Amounts of protoporphyrin IX (PpIX) accumulated in cells by 5-ALA loading (0, 0.0625, 0.125, 0.25, and 0.5 mmol/L) were measured using the fluorescence detection high-performance liquid chromatography method using four cell lines (ATN-1, TL-Om1, HuT102 and MT-1) derived from human adult T-cell leukemia/lymphoma (ATL) and one kind of HTLV-1 transformed cell line (C8166). Furthermore, as a cell killing assay by PDT, all cells were irradiated with light rays having a central wavelength of 630 nm (10, 30, and 100 J/cm2), and cell viability (%) were calculated with Cell Counting Kit-8 for quantitation of viable cell number. For the results of all cell lines, the cell viabilities were plotted on a vertical axis and the amounts of intracellular PpIX accumulation were plotted on a horizontal axis for each light irradiation energy density, and regression analysis was performed with statistical analysis software SAS software Release 9.3 (FIG. 3). As a result, these plots were converged to a four-parameter logistic model at all the light irradiation energy densities (Table 1), and it was recognized that cell death can be curvilinearly regressed by the intracellular PpIX accumulation and the light irradiation energy density without any differences among the cell lines, and it became clear that this relationship can be strictly defined.

[Table 1]

[Analysis Results]
    Model Equation: Y=(A-D)/(1+(X/C)^B)+D

| J/cm^2 | A | B | C | D | EC50 | 95%CI(lower) | 95%CI(upper) |
|---|---|---|---|---|---|---|---|
| 10 | 99.7265 | 1.0726 | 10.7995 | -17.9365 | 8.07358 | 6.60154 | 9.80959 |
| 30 | 99.6818 | 1.4497 | 3.6967 | -0.4281 | 3.66085 | 2.81588 | 4.75579 |
| 100 | 101.1 | 1.6317 | 2.1305 | 1.1648 | 2.18955 | 1.57317 | 3.06002 |

(Example 2)

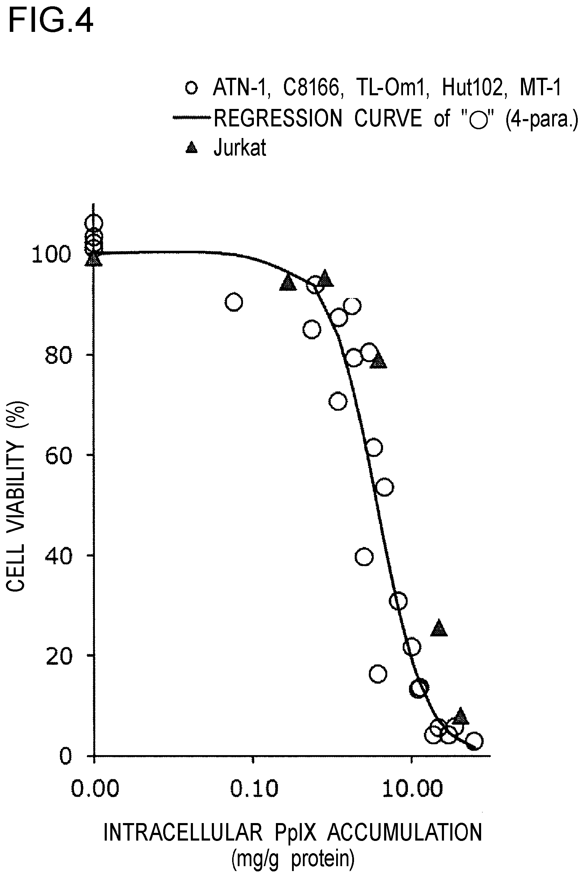

For Jurkat cells derived from human T-cell leukemia which is a disease classification different from that of the human adult T-cell leukemia/lymphoma clinically, amounts of protoporphyrin IX accumulated in the cells by 5-ALA loading (0, 0.0625, 0.125, 0.25, 0.5 and 1 mmol/L) were measured using the fluorescence detection high-performance liquid chromatography method, and then light irradiation (30 J/cm$^2$) with a central wavelength of 630 nm was further performed as the cell killing assay by PDT, and then cell viabilities (%) using the cell counting kit (Cell Counting Kit-8) were calculated. As a result, the cell viabilities were located on the regression curve described in FIG. 3, and it was proved that this relationship is strictly established even in leukemias having different disease classification (FIG. 4).

(Example 3)

(One aspect in clinical use)

After a cancer patient takes 5-ALAs, PpIX accumulation (X) accumulated in patient cancer cells is measured, and then the intracellular PpIX accumulation and a therapeutic target value of a killing effect (corresponding to cell viability (Y)) is input to a photodynamic therapy apparatus. The photodynamic therapy apparatus determines light irradiation energy density (P) corresponding to the therapeutic target value by referring to a regression curve programmed in advance from the input values or a lookup table having a stepwise range derived from the regression curve, and then performs therapy. At this time, the intracellular PpIX accumulation, the therapeutic target value of the killing effect, and the light irradiation energy density may be continuous values or stepwise values.

(Example 4)

(One aspect in clinical use)

The intracellular PpIX accumulation (X) to be treated is measured, and then this value is input to the photodynamic therapy apparatus. By referring to the regression curve programmed in advance from the input intracellular PpIX accumulation or the lookup table having a stepwise range derived from the regression curve, cell viability (Y) can be calculated for any light irradiation energy densities (P), and then therapeutic effect prediction or therapy is performed. The intracellular PpIX accumulation, the light irradiation energy density, and the cell viability may be continuous values or stepwise values.

(Example 5)

(One aspect in clinical use)

After one or more photodynamic therapies are administered to the patient using condition parameters (for example, light irradiation energy density) determined using the regression curve or the lookup table as described above, the cell viability of the patient is measured. From the light irradiation energy density and the cell viability at that time, the intracellular PpIX accumulation (X) or a range of the accumulation in the patient cancer cells is calculated based on the regression curve programmed in the photodynamic therapy apparatus.

(Example 6)

(One aspect in clinical use)

Furthermore, from the PpIX accumulation (X) calculated after the therapy is performed as described above, a medical worker can reset the light irradiation energy density (P) and the 5-ALA dose based on the regression curve programmed in the photodynamic therapy apparatus in order to obtain the target therapeutic effect when performing the next photodynamic therapy.

INDUSTRIAL APPLICABILITY

The present invention is industrially very useful in that the photodynamic therapy can be optimized.

EXPLANATORY NOTE

10    PROCESSOR

11    DATA INPUT UNIT

12    DATA STORAGE UNIT

13    DATA OUTPUT UNIT

20    LIGHT SOURCE

21    LIGHT INTENSITY CONTROL UNIT

**Claims**

1.   A method for determining light irradiation conditions in photodynamic therapy in which a subject is light-irradiated to inactivate cells with protoporphyrin IX accumulation after administration of 5-aminolevulinic acids to the subject, the method being **characterized by** the steps of:

   Measuring an accumulation of protoporphyrin IX in cells removed from the subject; and
   Determining light irradiation energy density using a predetermined relational expression based on the accumulation of protoporphyrin IX measured in the above step.

2.   The method according to Claim 1, wherein said relational expression is determined based on a regression curve representing a correlation among the three condition parameters of cell viability (Y), intracellular protoporphyrin IX accumulation (X), and light irradiation energy density (P), which are calculated in advance.

3.   The method according to Claim 1 or 2, wherein said relational expression includes a lookup table illustrating a relationship among the protoporphyrin IX accumulation (X) and the light irradiation energy density (P).

4.   The method according to any one of Claims 1 to 3, further including: determining a plurality of times of light irradiation when the measured protoporphyrin IX accumulation is less than a predetermined value.

5.   The method according to Claim 1, including steps of:

   calculating, in an experimental phase, a regression curve representing a correlation among three condition parameters: cell viability (Y), intracellular protoporphyrin IX accumulation (X), and light irradiation energy density (P); and
   selecting in advance, prior to initiation of therapy, two condition parameters from the cell viability (Y), the intracellular protoporphyrin IX accumulation (X), and the light irradiation energy density (P), and then determining the remaining condition parameter using the regression curve.

6.   The method according to Claim 5, wherein the regression curve is expressed by the following model equation using four parameters A to D.

$$Y = (A-D)/(1+(X/C)^B)+D$$

7. The method according to Claim 6, wherein the cell has a protoporphyrin IX-accumulating disease selected from a group consisting of leukemia, tumor, cancer, viral infection, skin disease, eye disease, autoimmune disease, and graft-versus-host disease (GVHD).

8. The method according to any one of Claims 5 to 7, further including steps of:

   Measuring cell viability of a subject after performing therapy using condition parameters determined using said regression curve, and estimating an intracellular protoporphyrin IX accumulation based on the obtained cell viability; and
   resetting, prior to initiation of the next therapy, the light irradiation energy density and the 5-aminolevulinic acid dose using the estimated intracellular protoporphyrin IX accumulation.

9. A photodynamic therapy apparatus comprising:

   a light source (20) for irradiating protoporphyrin IX accumulated in cells with light after administration of 5-aminolevulinic acids;
   a light intensity control unit (21) for controlling light irradiation energy density (P);
   a data input unit (11) for inputting data;
   a processor (10) for calculating data; and
   a data storage unit (12) for recording data,
   wherein the photodynamic therapy apparatus is **characterized in that** the processor (10) measures an accumulation of protoporphyrin IX in cells removed from the subject, and then determines light irradiation energy density using a predetermined relational expression based on the measured accumulation of protoporphyrin IX.

10. A photodynamic therapy apparatus comprising:

    a light source (20) for irradiating protoporphyrin IX accumulated in cells with light after administration of 5-aminolevulinic acids;
    a light intensity control unit (21) for controlling light irradiation energy density (P);
    a data input unit (11) for inputting data;
    a processor (10) for calculating data; and
    a data storage unit (12) for recording data,
    wherein the photodynamic therapy apparatus is **characterized in that** the processor (10) calculates, in an experimental phase, a regression curve representing a correlation among three condition parameters of cell viability (Y), intracellular protoporphyrin IX accumulation (X), and the light irradiation energy density (P), and the processor (10) selects in advance, prior to initiation of therapy, two condition parameters from the cell viability (Y), the intracellular protoporphyrin IX accumulation (X), and the light irradiation energy density (P), and determines the remaining condition parameter using the regression curve.

11. The photodynamic therapy apparatus according to Claim 9 or 10, wherein the regression curve is expressed by the following model equation using four parameters A to D.

$$Y = (A-D)/(1+(X/C)^B)+D$$

12. The photodynamic therapy apparatus according to Claim 9 or 10, wherein the cell has a protoporphyrin IX-accumulating disease selected from a group consisting of leukemia, tumor, cancer, viral infection, skin disease, eye disease, autoimmune disease, and graft-versus-host disease (GVHD).

13. A photodynamic therapy composition including 5-aminolevulinic acids having a dose corresponding to the intracellular protoporphyrin IX accumulation (X) determined by the method according to any one of Claims 5 to 7.

**Patentansprüche**

1. Verfahren zur Bestimmung von Lichtbestrahlungsbedingungen bei einer photodynamischen Therapie, bei der ein Subjekt mit Licht bestrahlt wird, um Zellen mit Protoporphyrin IX-Akkumulation nach Verabreichung von 5-Aminolävulinsäuren an das Subjekt zu inaktivieren, wobei das Verfahren **gekennzeichnet ist durch** die Schritte eines:

Messens einer Akkumulation von Protoporphyrin IX in dem Subjekt entnommenen Zellen, und
Bestimmens einer Lichtbestrahlungsenergiedichte unter Verwendung eines vorbestimmten relationalen Ausdrucks auf der Grundlage der im obigen Schritt gemessenen Akkumulation von Protoporphyrin IX.

2.  Verfahren nach Anspruch 1, wobei der relationale Ausdruck auf der Grundlage einer Regressionskurve bestimmt ist, die eine Korrelation zwischen den drei Zustandsparametern der Zelllebensfähigkeit (Y), der intrazellulären Protoporphyrin IX-Akkumulation (X) und der Lichtbestrahlungsenergiedichte (P) darstellt, die im Voraus berechnet werden.

3.  Verfahren nach Anspruch 1 oder 2, wobei der relationale Ausdruck eine Lookup-Tabelle umfasst, die eine Beziehung zwischen der Protoporphyrin IX-Akkumulation (X) und der Lichtbestrahlungsenergiedichte (P) darstellt.

4.  Verfahren nach einem der Ansprüche 1 bis 3, ferner mit: mehrfaches Bestimmen der Lichtbestrahlung, wenn die gemessene Protoporphyrin IX-Akkumulation kleiner als ein vorbestimmter Wert ist.

5.  Verfahren nach Anspruch 1, mit den Schritten eines:

    Berechnens einer Regressionskurve in einer experimentellen Phase, die eine Korrelation zwischen drei Zustandsparametern einer Zelllebensfähigkeit (Y), einer intrazellulären Protoporphyrin IX-Akkumulation (X) und einer Lichtbestrahlungsenergiedichte (P) darstellt, und
    Auswählens von zwei Zustandsparametern aus der Zelllebensfähigkeit (Y), der intrazellulären Protoporphyrin IX-Akkumulation (X) und der Lichtbestrahlungsenergiedichte (P) im Voraus, bevor eine Therapie eingeleitet wird, und anschließenden Bestimmens des verbleibenden Zustandsparameters unter Verwendung der Regressionskurve.

6.  Verfahren nach Anspruch 5, wobei die Regressionskurve durch die folgende Modellgleichung unter Verwendung von vier Parametern A bis D ausgedrückt wird:

$$Y = (A-D)/(1+(X/C)^B)+D$$

7.  Verfahren nach Anspruch 6, wobei die Zelle eine Protoporphyrin IX-akkumulierende Krankheit aufweist, ausgewählt aus einer Gruppe bestehend aus Leukämie, Tumor, Krebs, Virusinfektion, Hautkrankheit, Augenkrankheit, Autoimmunerkrankung und Graft-versus-Host-Reaktion (GVHD).

8.  Verfahren nach einem der Ansprüche 5 bis 7, ferner mit Schritten eines:

    Messens der Zelllebensfähigkeit eines Subjekts nach Durchführung der Therapie unter Verwendung von Zustandsparametern, die unter Verwendung der Regressionskurve bestimmt werden, und Schätzens einer intrazellulären Protoporphyrin IX-Akkumulation auf der Grundlage der erhaltenen Zelllebensfähigkeit, und
    Zurücksetzens der Lichtbestrahlungsenergiedichte und der 5-Aminolävulinsäure-Dosis unter Verwendung der geschätzten intrazellulären Protoporphyrin IX-Akkumulation vor Beginn der nächsten Therapie.

9.  Photodynamische Therapievorrichtung, mit:

    einer Lichtquelle (20) zum Bestrahlen von in Zellen akkumuliertem Protoporphyrin IX mit Licht nach Verabreichung von 5-Aminolävulinsäuren,
    einer Lichtintensitätssteuereinheit (21) zum Steuern einer Lichtbestrahlungsenergiedichte (P),
    einer Dateneingabeeinheit (11) zum Eingeben von Daten,
    einem Prozessor (10) zum Berechnen von Daten und
    einer Datenspeichereinheit (12) zum Aufzeichnen von Daten,
    wobei die photodynamische Therapievorrichtung **dadurch gekennzeichnet ist, dass** der Prozessor (10) eine Akkumulation von Protoporphyrin IX in dem Subjekt entnommenen Zellen misst und dann die Lichtbestrahlungsenergiedichte unter Verwendung eines vorbestimmten relationalen Ausdrucks auf der Grundlage der gemessenen Akkumulation von Protoporphyrin IX bestimmt.

10. Vorrichtung zur photodynamischen Therapie, mit:

    einer Lichtquelle (20) zum Bestrahlen von in Zellen akkumuliertem Protoporphyrin IX mit Licht nach Verabreichung von 5-Aminolävulinsäuren,

einer Lichtintensitätssteuereinheit (21) zum Steuern einer Lichtbestrahlungsenergiedichte (P),

einer Dateneingabeeinheit (11) zum Eingeben von Daten,

einem Prozessor (10) zum Berechnen von Daten und

einer Datenspeichereinheit (12) zum Aufzeichnen von Daten,

wobei die photodynamische Therapievorrichtung **dadurch gekennzeichnet ist, dass** der Prozessor (10) in einer experimentellen Phase eine Regressionskurve berechnet, die eine Korrelation zwischen drei Zustandsparametern einer Zelllebensfähigkeit (Y), einer intrazellulären Protoporphyrin IX-Akkumulation (X) und einer Lichtbestrahlungsenergiedichte (P) darstellt, und

der Prozessor (10) im Voraus zwei Zustandsparameter aus der Zelllebensfähigkeit (Y), der intrazellulären Protoporphyrin IX-Akkumulation (X), und der Lichtbestrahlungsenergiedichte (P) auswählt, bevor eine Therapie eingeleitet wird, und den verbleibenden Zustandsparameter unter Verwendung der Regressionskurve bestimmt.

11. Photodynamische Therapievorrichtung nach Anspruch 9 oder 10, wobei die Regressionskurve durch die folgende Modellgleichung unter Verwendung von vier Parametern A bis D ausgedrückt wird:

$$Y = (A-D)/(1+(X/C)^B)+D$$

12. Photodynamische Therapievorrichtung nach Anspruch 9 oder 10, wobei die Zelle eine Protoporphyrin IX-akkumulierende Krankheit aufweist, ausgewählt aus einer Gruppe bestehend aus Leukämie, Tumor, Krebs, Virusinfektion, Hautkrankheit, Augenkrankheit, Autoimmunerkrankung und Graft-versus-Host-Reaktion (GVHD).

13. Photodynamische Therapiezusammensetzung mit 5-Aminolävulinsäuren mit einer Dosis, die der intrazellulären Protoporphyrin IX-Akkumulation (X) entspricht, die durch das Verfahren nach einem der Ansprüche 5 bis 7 bestimmt wurde.

**Revendications**

1. Méthode pour déterminer les conditions d'irradiation lumineuse dans la thérapie photodynamique dans laquelle un sujet est irradié pour inactiver les cellules avec une accumulation de protoporphyrine IX après l'administration d'acides 5-aminolévuliniques au sujet, la méthode étant **caractérisée par** les étapes :

mesurer l'accumulation de protoporphyrine IX dans les cellules prélevées sur le sujet ; et
déterminer la densité énergétique de l'irradiation lumineuse à l'aide d'une expression relationnelle prédéterminée sur la base de l'accumulation de protoporphyrine IX mesurée à l'étape précédente.

2. Méthode selon la revendication 1, dans laquelle l'expression relationnelle est déterminée sur la base d'une courbe de régression représentant une corrélation entre les trois paramètres de viabilité cellulaire (Y), accumulation intracellulaire de protoporphyrine IX (X) et densité d'énergie d'irradiation lumineuse (P), qui sont calculés à l'avance.

3. Méthode selon la revendication 1 ou 2, dans laquelle ladite expression relationnelle comprend une table de recherche illustrant une relation entre l'accumulation de protoporphyrine IX (X) et la densité d'énergie d'irradiation lumineuse (P).

4. Méthode selon l'une des revendications 1 à 3, comprenant en outre : déterminer d'une pluralité de temps d'irradiation lumineuse lorsque l'accumulation de protoporphyrine IX mesurée est inférieure à une valeur prédéterminée.

5. Méthode selon la revendication 1, comprenant les étapes de :

calculer, dans une phase expérimentale, une courbe de régression représentant une corrélation entre trois paramètres d'état : viabilité cellulaire (Y), accumulation intracellulaire de protoporphyrine IX (X), et densité d'énergie d'irradiation lumineuse (P) ; et
sélectionner à l'avance, avant le début de la thérapie, deux paramètres d'état parmi la viabilité cellulaire (Y), l'accumulation intracellulaire de protoporphyrine IX (X) et la densité d'énergie d'irradiation lumineuse (P), puis déterminer le paramètre d'état restant à l'aide de la courbe de régression.

6. Méthode selon la revendication 5, dans laquelle la courbe de régression est exprimée par l'équation modèle suivante utilisant quatre paramètres A à D.

$$Y = (A-D)/(1+(X/C)^B)+D$$

7. Méthode selon la revendication 6, dans laquelle la cellule est atteinte d'une maladie accumulant la protoporphyrine IX, choisie dans le groupe constitué par la leucémie, la tumeur, le cancer, l'infection virale, la maladie de la peau, la maladie des yeux, la maladie auto-immune et la maladie du greffon contre l'hôte (GVHD).

8. Méthode selon l'une des revendications 5 à 7, comprenant en outre les étapes :

mesurer la viabilité cellulaire d'un sujet après l'exécution du traitement en utilisant les paramètres de condition déterminés à l'aide de ladite courbe de régression, et estimer une accumulation intracellulaire de protoporphyrine IX sur la base de la viabilité cellulaire obtenue ; et
réinitialiser, avant le début de la thérapie suivante, la densité d'énergie d'irradiation lumineuse et la dose d'acide 5-aminolévulinique en utilisant l'accumulation intracellulaire estimée de protoporphyrine IX.

9. Appareil de thérapie photodynamique comprenant :

une source de lumière (20) pour irradier la protoporphyrine IX accumulée dans les cellules avec de la lumière après l'administration d'acides 5-aminolévuliniques ;
une unité de contrôle de l'intensité lumineuse (21) pour contrôler la densité énergétique de l'irradiation lumineuse (P) ;
une unité d'entrée de données (11) pour l'entrée de données ;
un processeur (10) pour le calcul des données ; et
une unité de stockage de données (12) pour enregistrer les données,
l'appareil de thérapie photodynamique étant **caractérisé en ce que** le processeur (10) mesure l'accumulation de protoporphyrine IX dans les cellules prélevées sur le sujet, puis détermine la densité d'énergie d'irradiation lumineuse à l'aide d'une expression relationnelle prédéterminée basée sur l'accumulation mesurée de protoporphyrine IX.

10. Appareil de thérapie photodynamique comprenant

une source de lumière (20) pour irradier la protoporphyrine IX accumulée dans les cellules avec de la lumière après l'administration d'acides 5-aminolévuliniques ;
une unité de contrôle de l'intensité lumineuse (21) pour contrôler la densité énergétique de l'irradiation lumineuse (P) ;
une unité d'entrée de données (11) pour l'entrée de données ;
un processeur (10) pour le calcul des données ; et
une unité de stockage de données (12) pour enregistrer les données,
dans lequel l'appareil de thérapie photodynamique est **caractérisé en ce que** le processeur (10) calcule, dans une phase expérimentale, une courbe de régression représentant une corrélation entre trois paramètres de condition de viabilité cellulaire (Y), accumulation intracellulaire de protoporphyrine IX (X), et densité d'énergie d'irradiation lumineuse (P), et
le processeur (10) sélectionne à l'avance, avant le début de la thérapie, deux paramètres de condition parmi la viabilité cellulaire (Y), l'accumulation intracellulaire de protoporphyrine IX (X), et la densité d'énergie d'irradiation lumineuse (P), et détermine le paramètre de condition restant à l'aide de la courbe de régression.

11. Appareil de thérapie photodynamique selon la revendication 9 ou 10, dans lequel la courbe de régression est exprimée par l'équation modèle suivante utilisant quatre paramètres A à D.

$$Y = (A-D)/(1+(X/C)^B)+D$$

12. Appareil de thérapie photodynamique selon la revendication 9 ou 10, dans lequel la cellule est atteinte d'une maladie accumulant la protoporphyrine IX, choisie dans un groupe comprenant la leucémie, la tumeur, le cancer, l'infection virale, la maladie de la peau, la maladie des yeux, la maladie auto-immune et la maladie du greffon contre l'hôte (GVHD).

**13.** Composition de thérapie photodynamique comprenant des acides 5-aminolévuliniques ayant une dose correspondant à l'accumulation intracellulaire de protoporphyrine IX (X) déterminée par la méthode selon l'une des revendications 5 à 7.

# FIG.1

# FIG.2

```
            ┌──────────────┐
            │    START     │
            └──────────────┘
                   │
    ┌─────────────────────────────┐  s1
    │      MEASUREMENT OF          │
    │     INTRACELLULAR PpIX       │
    │  ACCUMULATION IN ANY CELLS   │
    └─────────────────────────────┘
                   │
    ┌─────────────────────────────┐  s2
    │         REGRESSION           │
    │          ANALYSIS            │
    │         AND FITTING          │
    └─────────────────────────────┘
                   │
  ┌───────────────────────────────────┐  s3
  │   DETERMINATION OF LIGHT          │
  │  IRRADIATION ENERGY DENSITY,      │
  │ AMOUNT OF IRRADIATION, NUMBER     │
  │    OF IRRADIATION TIMES,          │
  │    DOSE OF 5-ALA, ETC.            │
  └───────────────────────────────────┘
                   │
            ┌──────────────┐
            │     END      │
            └──────────────┘
```

## FIG.3

IRRADIATION at 10 J/cm$^2$     IRRADIATION at 30 J/cm$^2$     IRRADIATION at 100 J/cm$^2$

CELL VIABILITY (%)

INTRACELLULAR PpIX ACCUMULATION (10x, mg/g protein)

EP 3 928 831 B1

# FIG.4

○ ATN-1, C8166, TL-Om1, Hut102, MT-1
——REGRESSION CURVE of "○" (4-para.)
▲ Jurkat

INTRACELLULAR PpIX ACCUMULATION
(mg/g protein)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060282132 A1
- US 20160361418 A1
- US 20050112131 A1
- US 20170042938 A1

- WO 2015125732 A
- JP 2017526371 A
- JP 5612246 B
- US 6609014 B

**Non-patent literature cited in the description**

- **OUYANG G. et al.** Inhibition of autophagy potentiates the apoptosis-inducing effects of photodynamic therapy on human colon cancer cells. *Photodiagnosis Photodyn Ther.*, 2018, vol. 21, 396-403